Europäisches Patentamt

European Patent Office

Office européen des brevets

(11) Numéro de publication: **0 216 706 B1**

(19)

(12) **FASCICULE DE BREVET EUROPEEN**

(45) Date de publication de fascicule du brevet: **13.05.92** (51) Int. Cl.⁵: **G01N 7/00**

(21) Numéro de dépôt: **86402077.1**

(22) Date de dépôt: **23.09.86**

(54) **Procédé et dispositif de mesure du point de bulle du pétrole d'une formation souterraine.**

(30) Priorité: **23.09.85 FR 8514060**

(43) Date de publication de la demande:
**01.04.87 Bulletin 87/14**

(45) Mention de la délivrance du brevet:
**13.05.92 Bulletin 92/20**

(84) Etats contractants désignés:
**DE FR GB IT NL**

(56) Documents cités:
**FR-A- 2 474 196**
**FR-A- 2 476 205**
**FR-A- 2 480 941**
**GB-A- 1 601 997**
**US-A- 2 138 141**

(73) Titulaire: **FLOPETROL SERVICES, INC.**
**8, Aquilino de la Guardia**
**Panama City(PA)**

(72) Inventeur: **Glotin, Bernard J.P.**
**78, avenue du Bois Guimier**
**F-94000 Saint-Maur(FR)**
Inventeur: **Exbrayat, Jacques**
**19, rue Rosa Bonheur**
**F-77000 Melun(FR)**

(74) Mandataire: **Hagel, Francis et al**
**Etudes et Productions Schlumberger A L'AT-**
**TENTION DU SERVICE BREVETS 26, rue de la**
**Cavée B.P. 202**
**F-92142 Clamart Cédex(FR)**

Rank Xerox (UK) Business Services
(3.08/2.19/2.0)

## Description

La présente invention se rapporte à la mesure du point de bulle du pétrole d'une formation souterraine dans laquelle a été foré un puits.

On sait que, pour étudier les caractéristiques du pétrole contenu dans une formation souterraine, on en prélève des échantillons que l'on fait remonter jusqu'à la surface. Toutefois, pour que le pétrole ainsi prélevé dans le puits ait gardé les propriétés qu'il présente loin du puits au sein de ladite formation, il est nécessaire que son état soit monophasique à l'endroit de prélèvement; sinon, les échantillons ne seraient pas représentatifs de l'ensemble du pétrole de la formation.

Habituellement, on s'assure de l'état monophasique du pétrole prélevé dans le puits en mesurant le point de bulle des échantillons remontés à la surface, c'est-a-dire la pression au delà de laquelle les gaz mélangés au pétrole y sont entièrement dissous, de sorte que le pétrole se présente comme un liquide monophasique. Connaissant la pression et la température qui règnent au fond du puits, à l'endroit du prélèvement, on en déduit si le pétrole qui s'y trouve est à l'état monophasique. Pareille mesure du point de bulle, effectué en laboratoire, exige une recompression du pétrole à une pression de l'ordre ce celle qui règne dans le puits, ainsi qu'un chauffage à la température du gisement souterrain.

Un tel mode de mesure nécessite des opérations longues, dont les résultats concernant la validité des échantillons ne sont connus que bien après leur prélèvement, alors qu'on ne peut même parfois envisager de nouveaux prélèvements.

Le brevet FR-A-2 476 205 décrit un procédé et un dispositif d'étude hydrodynamique d'un puits. Dans cette technique on prélève un échantillon de fluide de volume plus petit que la chambre d'échantillonnage, on ferme hermétiquement l'échantillon et l'on fait augmenter son volume. Pendant l'augmentation de volume, on enregistre la pression et l'accroissement de volume afin d'obtenir le point de bulle du fluide échantillonné. Dans la chambre, la pression est toujours inférieure à la valeur à laquelle a été prélevé l'échantillon.

L'invention a pour but de remédier aux inconvénients précédents, en effectuant directement dans le puits une mesure rapide permettant de s'assurer de la validité des échantillons prélevés.

Selon un premier aspect de l'invention, afin d'obtenir une mesure précise même dans le cas où la pression du point de bulle est voisine de la pression régnant dans le puits, un procédé de mesure du point de bulle du pétrole d'une formation souterraine dans laquelle a été foré un puits comprend les étapes de la revendication 1.

Selon un deuxième aspect de l'invention, un dispositif de mesure du point de bulle du pétrole d'une formation souterraine dans laquelle a été foré un puits comprend l'outil de la revendication 2.

Dans une forme d'exécution préférée, l'aiguille mobile du dispositif comporte deux parties successives de section différente, savoir une première partie qui se prolonge par une deuxième partie de section inférieure à celle de la première, tandis que la chambre possède deux orifices alignés permettant à l'aiguille de traverser de part en part la chambre, savoir un premier orifice de section conjuguée de celle de la première partie de l'aiguille et un deuxième orifice de section conjuguée de celle de la deuxième partie, ces orifices étant obturés respectivement par lesdites parties de l'aiguille lorsque celle-ci est amenée à traverser la chambre. On conçoit que, dans ce cas, cette dernière se trouve complètement isolée du milieu extérieur et que la petite quantité de pétrole qui s'y trouve confinée est soumise à des variations de pression résultant des variations de volume créées par les déplacements de l'aiguille, le volume offert au pétrole dépendant de la proportion variable entre les longueurs respectives de sa première et de sa deuxième partie qui se trouvent comprises à l'intérieur de la chambre. Lorsque les moyens d'entraînement de l'aiguille la font se retirer complètement de la chambre en libérant l'orifice ou les orifices qu'elle comporte, la chambre est mise en communication avec le milieu extérieur et se remplit de pétrole.

De préférence, on prévoit une première valve tarée et une deuxième valve tarée de limitation de pression entre le volume intérieur de la chambre et le milieu extérieur, pour limiter respectivement la surpression et la dépression pouvant apparaître dans la chambre, au cours des déplacements de l'aiguille, par rapport à la pression régnant dans le puits.

Les moyens d'entraînement de l'aiguille peuvent comprendre un moteur électrique logé dans un carter étanche à l'épreuve de la pression, une tige filetée entraînée en rotation par le moteur à travers la paroi de son carter grâce à un dispositif d'accouplement magnétique, et un écrou monté sur la tige filetée et solidaire de l'aiguille, qu'il anime de mouvements de translation longitudinale lorsque le moteur fait tourner la tige filetée. Quant au capteur de déplacement mesurant les mouvements de l'aiguille, il peut être constitué par un potentiomètre linéaire.

Lorsque l'orifice de la chambre avec lequel est susceptible de coopérer la deuxième partie de l'aiguille est muni d'un joint annulaire d'étanchéité, il convient, pour éviter tout endommagement ou perte de ce joint au moment où la chambre est réouverte sur le milieu extérieur via cet orifice, de prévoir, creusé dans l'extrémité de l'aiguille, un conduit d'égalisation de pression mettant en communication, quand l'aiguille, au cours d'un mouvement de retrait de la chambre, va quitter ledit orifice, la chambre avec le milieu extérieur alors que l'extrémité de l'aiguille est encore engagée dans le joint.

L'invention décrite dans ce qui précède permet d'obtenir, directement dans le puits, une bonne approximation du point de bulle du pétrole qu'il contient, ce qui permet de valider tout échantillon prélevé dans le puits et de bénéficier d'un contrôle de la qualité des échantillons sur le site même d'exploitation. Si pour une raison quelconque l'échantillon n'était pas validé, on pourrait recommencer les opérations de prise d'échantillon et de validation du nouvel échantillon prélevé lorsque le dispositif de mesure est encore dans le puits.

D'autres caractéristiques et avantages de l'invention ressortiront plus clairement de la description qui va suivre, en regard des dessins annexés, d'un exemple de réalisation non limitatif.

La figure 1 représente schématiquement, en coupe verticale, un puits de pétrole dans lequel a été descendu un dispositif de mesure selon l'invention.

La figure 2 représente une courbe pression/volume permettant de déterminer le point de bulle d'un mélange liquide-gaz.

La figure 3 représente, schématiquement et de manière simplifiée, la structure d'un dispositif de mesure selon l'invention.

Les figures 4a à 4d illustrent schématiquement les phases successives d'une opération de mesure du point de bulle selon l'invention.

Les figures 5 à 9 représentent en coupe longitudinale différentes parties d'un dispositif selon l'invention, réparties sur sa longueur, suivant un exemple concret de réalisation.

La figure 10 représente une coupe transversale suivant la ligne X-X de la figure 6.

La figure 11 représente schématiquement la configuration du potentiomètre linéaire constituant un capteur de déplacement.

La figure 12 représente une coupe transversale suivant la ligne XII-XII de la figure 7.

La figure 13 représente, à échelle légèrement agrandie, une coupe longitudinale de l'extrémité de l'aiguille mobile appartenant au dispositif, montrant le conduit d'égalisation de pression.

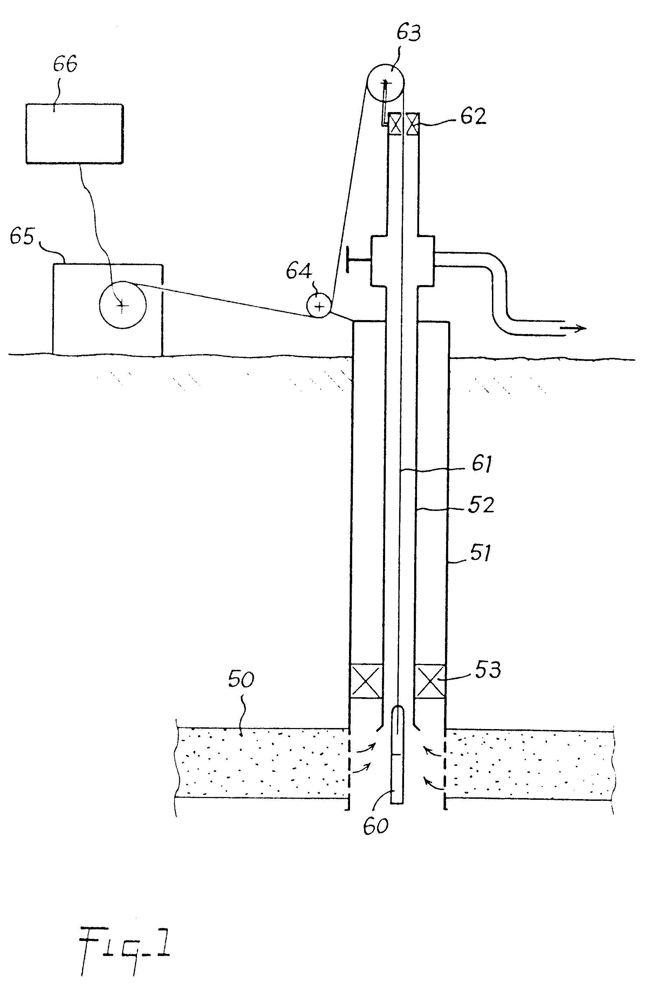

On voit sur la figure 1 un puits creusé dans une zone 50 productrice de pétrole et comportant, dans un tubage 51, une colonne de production 52. Entre l'extrémité inférieure de cette dernière et le tubage 51 est placé un dispositif d'étanchéité annulaire 53. Un dispositif de mesure 60 selon l'invention est descendu au niveau de la zone productrice 50 à l'aide d'un câble 61 auquel il est suspendu.

Ce câble, passant à l'intérieur de la colonne de production 52, en émerge à son sommet à travers un dispositif d'étanchéité 62 pour aller s'enrouler, via des poulies de renvoi 63, 64, sur le tambour d'un treuil 65 disposé à la surface du sol. Le câble 61 est un câble électrique qui, outre une fonction mécanique de suspension du dispositif de mesure 60, assure une fonction de transmission de signaux de mesure vers la surface et de signaux de commande vers le dispositif 60, ainsi que vers d'autres dispositifs qui peuvent être associés à ce dernier, notamment des capacités d'échantillonnage dont le déclenchement est commandé depuis la surface. Ces signaux sont gérés par un calculateur 66 constituant une unité de commande et d'enregistrement.

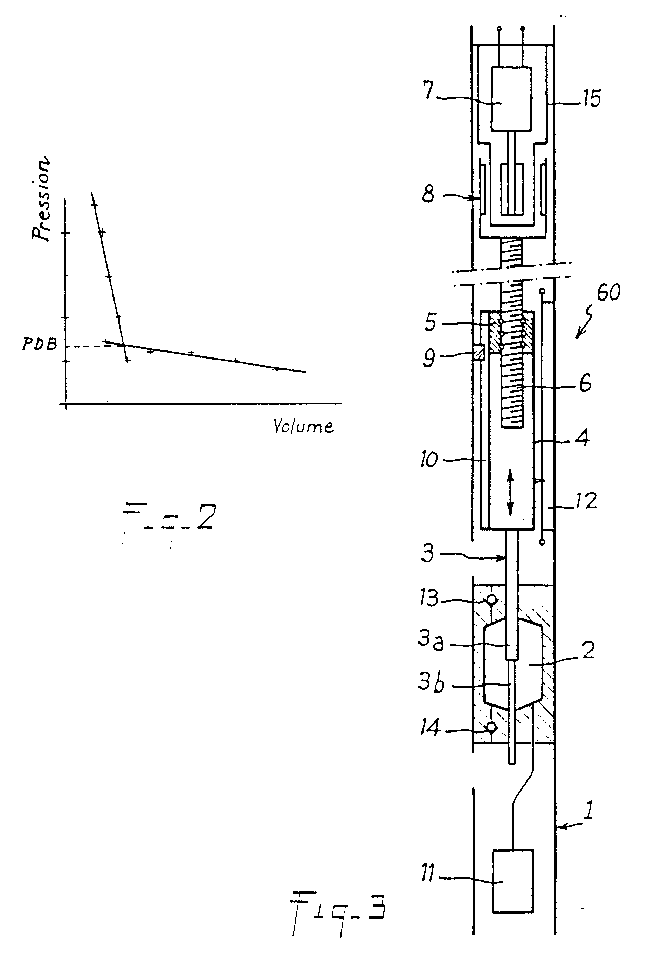

Le fonctionnement du dispositif de mesure 60 repose sur le diagramme de la figure 2 montrant les variations de la pression en fonction du volume d'un mélange liquide-gaz, le gaz offrant une certaine solubilité dans le liquide. Lorsque la pression appliquée au mélange est élevée, une variation de pression donnée entraîne une faible variation de volume du mélange. Toutefois, si la pression s'abaisse au-dessous d'une valeur PDB, des bulles de gaz apparaissent dans le mélange et le volume varie beaucoup plus pour une même variation de pression. La pression PDB est le "point de bulle", au-dessus duquel on est sûr que le mélange est complètement liquide et ne renferme aucune fraction à l'état gazeux.

C'est à la mesure du point de bulle d'un mélange d'hydrocarbures et de gaz qu'est destiné le dispositif que l'on va décrire, cette mesure devant être effectuée au fond d'un puits foré dans une formation souterraine, préalablement à une prise d'échantillon du mélange fluide.

Conformément à la figure 3, l'appareil comprend essentiellement une longue enveloppe 1 en forme de cylindre de révolution, une chambre 2 à paroi épaisse, à l'intérieur de laquelle peut être introduite une aiguille 3 possédant deux parties 3a, 3b de diamètre différent, cette aiguille pouvant être animée de mouvements de translation longitudinale par un mécanisme composé d'une pièce cylindrique 4 à une extrémité de laquelle est fixée l'aiguille 3 et qui est solidaire à son autre extrémité d'un écrou 5 monté sur une tige filetée 6 entraînée en rotation par un moteur électrique 7 placé dans un carter étanche 15, par l'intermédiaire d'un dispositif d'accouplement magnétique 8. Au-dessus du moteur 7 se trouve la partie électronique de commande de l'outil, ainsi qu'un connecteur qui permet de brancher le dispositif soit directement à un câble électrique remontant jusqu'en surface, soit à un autre dispositif. Lorsque le moteur 7 est mis en marche, dans un sens ou dans l'autre, il fait tourner la tige filetée 6, de sorte que l'écrou 5 se déplace suivant la direction longitudinale de celle-ci, entraînant en translation la pièce 4, laquelle est empêchée de tourner par un ergot 9 fixe engagé dans une rainure de guidage rectiligne 10 appartenant à la pièce 4, cette dernière faisant pénéter dans la chambre 2 l'aiguille 3 ou l'en faisant ressortir suivant le sens de rotation du moteur 7. Selon le degré d'engagement de la partie 3a de plus grand diamètre de l'aiguille 3, le volume intérieur de la chambre 2 varie, ce qui induit des variations de la pression à laquelle est soumis le fluide contenu dans ladite chambre, qui est mesurée à l'aide d'un manomètre 11. Le volume de la chambre 2 se déduit de la position longitudinale de la pièce 4 portant l'aiguille 3, connue grâce à un potentiomètre linéaire 12 mesurant les déplacements de ladite pièce par rapport à l'enveloppe 1 du dispositif.

Le dispositif comporte également une sonde thermométrique permettant de mesurer la température du pétrole dans le puits. Les valeurs mesurées par cette sonde sont transmises à la surface, comme celles que délivrent le manomètre 11 et le potentiomètre 12.

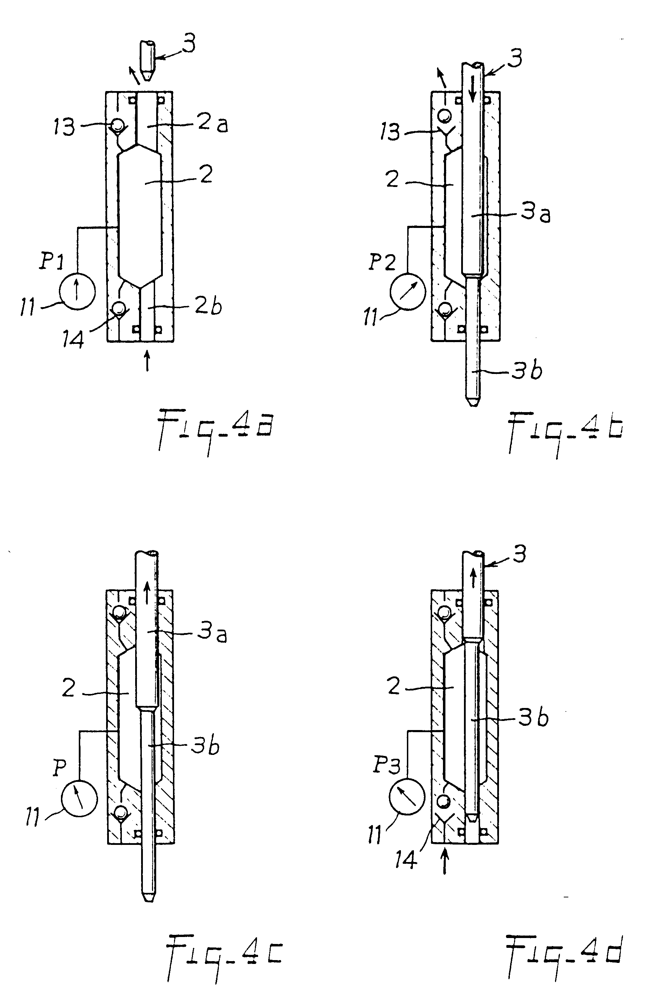

Les principales phases opératoires d'une mesure de point de bulle vont maintenant être expliquées à l'aide des figures 4a à 4d.

Initialement (figure 4a), l'aiguille 3 est complètement retirée de la chambre 2, libérant les orifices 2a, 2b pratiqués en alignement dans la paroi de la chambre 2 pour permettre le passage de l'aiguille. Le fluide objet de la mesure peut alors passer librement à travers la chambre 2, soit par circulation naturelle du fluide au niveau du dispositif, soit grâce à un déplacement vertical imprimé à ce dernier dans le puits où il a été descendu. La chambre 2 se remplit alors d'une petite quantité du fluide, sous la pression P1 régnant dans le puits.

Puis, mettant en marche le moteur 7, on fait pénétrer l'aiguille 3 dans la chambre 2. Les orifices 2a et 2b, dont le diamètre est respectivement égal au diamètre des parties 3a et 3b de l'aiguille, sont alors obturés de façon étanche par ces dernières, et, le fluide présent dans la chambre 2 s'y trouve confiné sous un certain volume. Puis l'aiguille 3 continuant sa course, sa partie 3a pénètre dans la chambre et fait diminuer le volume que celle-ci offre au fluide (figure 4b), lequel est par suite soumis à une pression croissante, supérieure à P1. La valeur de cette pression est limitée à une valeur maximale $P2 = P1 + \Delta P$ par une valve de décharge tarée 13 connectée entre le volume intérieur de la chambre 2 et l'espace extérieur à la pression P1.

L'aiguille 3 ayant atteint sa position d'enfoncement maximal où la portion contenue dans la chambre 2 appartient à peu près exclusivement à sa partie 3a de plus grand diamètre, le moteur 7 est arrêté, puis remis en marche pas à pas dans l'autre sens, de façon qu'il cause une extraction par paliers de l'aiguille 3, d'où résulte une augmentation graduelle du volume de la chambre 2, la partie 3a y cédant peu à peu la place à la partie 3b de diamètre inférieur. Toutefois, cette dernière partie présente une longueur suffisante pour maintenir obturé l'orifice 2b qu'elle traverse, et cela sensiblement jusqu'à ce que la partie 3a ait complètement quitté la chambre 2. Concomitamment, la pression P du fluide va en diminuant, et elle est mesurée par le manomètre 11 tandis que le volume croissant de la chambre est déterminé à partir de la position de l'aiguille 3 mesurée par le potentiomètre 12. La pression P descend ainsi jusqu'à une valeur limitée inférieurement par une seconde valve de décharge 14, disposée comme la valve 13, mais en sens inverse, savoir à une valeur minimale $P3 = P1 - \Delta P'$ inférieure à la pression P1, mais présentant avec celle-ci un écart $\Delta P'$ non excessif.

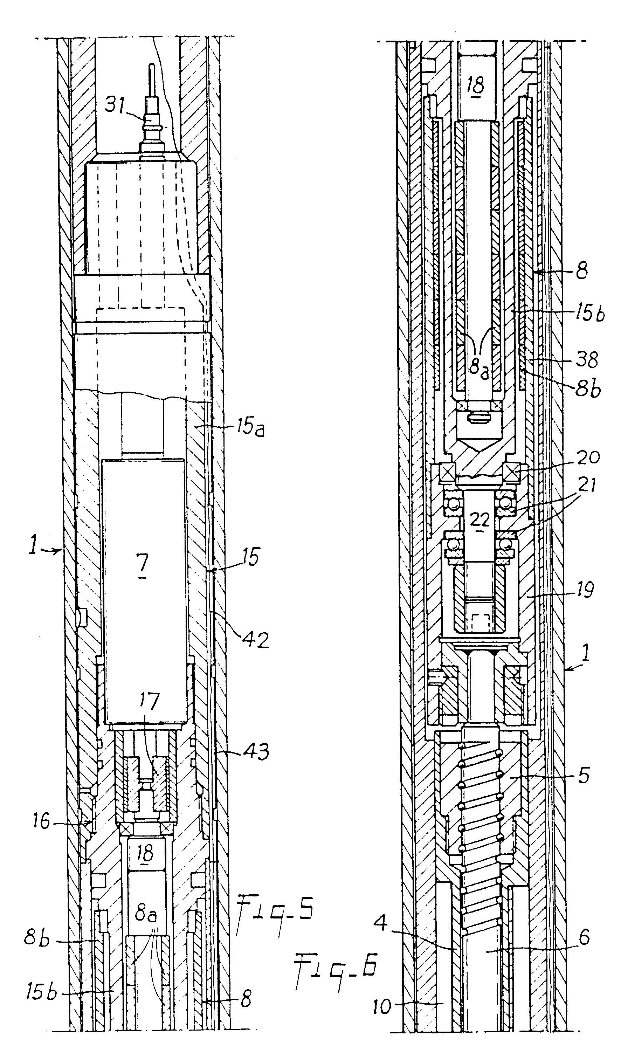

Les figures 5 à 9 montrent un exemple concret de réalisation d'un dispositif selon l'invention. Elles font voir la structure interne de portions du dispositif qui se succèdent, suivant l'ordre de numérotation des figures, de haut en bas lorsque le dispositif est en place dans la colonne de production d'un puits de pétrole.

On y reconnaît :

- le moteur 7 et son carter étanche 15, formé de deux parties 15a et 15b réunies par vissage en 16, la partie 15a comportant des traversées électriques étanches 31 permettant l'alimentation du moteur;
- le dispositif d'accouplement magnétique 8, composé d'un arbre central 18 entraîné par le moteur 7 par l'intermédiaire d'un joint souple 17 et portant des aimants 8a, et, à l'extérieur du carter 15, d'un élément métallique tubulaire 38 disposé coaxialement à l'arbre 18 et portant des aimants 8b. Le carter 15 est réalisé en alliage amagnétique de façon que les champs des aimants 8a, 8b puissent le traverser pour assurer la transmission d'un effort mécanique de l'arbre 18 à l'élément 38. Ce dernier se prolonge par une pièce tubulaire de portée 19 qui est guidée en rotation sans possibilité de translation par rapport à l'axe général du dispositif par un roulement à billes 20 et des butées à billes 21 montés sur un arbre court 22 fixe, solidaire du carter 15;
- la tige filetée 6, solidaire de l'élément tubulaire 8b par l'intermédiaire de la pièce de portée 19;
- l'écrou 5, qui est un écrou à billes, monté sur la tige filetée 6;

- la pièce cylindrique 4, solidaire de l'écrou 5 et entraînée par celui-ci en translation suivant l'axe longitudinal du dispositif, sans possibilité de rotation grâce à sa rainure de guidage 10 dans laquelle est engagé l'ergot fixe 9;
- l'aiguille 3, solidaire de la pièce 4 et offrant deux parties 3a, 3b de diamètre différent;
- la chambre 2 où peut pénétrer et que peut traverser l'aiguille 3 par des orifices 2a, 2b dotés de joints toriques d'étanchéité 23, 24 et respectivement adaptés aux diamètres des parties 3a et 3b de l'aiguille 3;
- le manomètre 11, relié, via un conduit 30, par un tube capillaire 25 à la chambre 2, ce tube, rempli d'un fluide neutre, ayant pour rôle de protéger les éléments internes du manomètre.

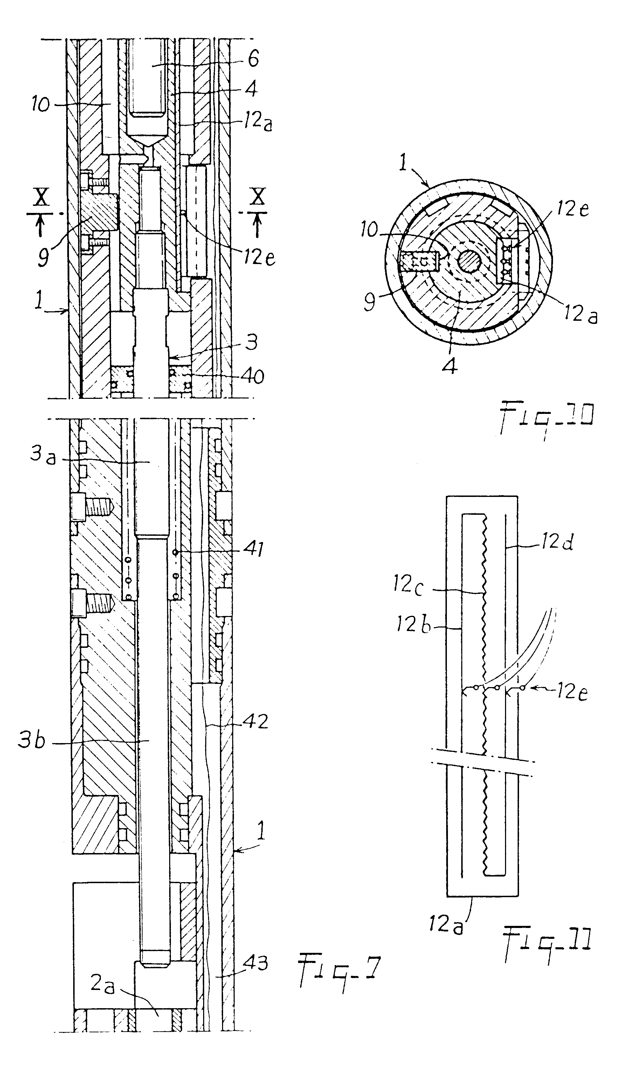

L'espace intérieur du dispositif qui s'étend entre les traversées électriques étanches 31 situées au-dessus du moteur 7 (figure 5) et des traversées électriques étanches prévues dans une zone 39 située à l'autre extrémité du dispositif (figure 9) est rempli d'un fluide isolant (huile minérale). Selon un mode de construction traditionnel, ce fluide est maintenu à la même pression que celle du puits au moyen d'un piston flottant 40 (figure 7) assurant la séparation entre l'affluent du puits et le fluide isolant interne au dispositif. Le piston de séparation 40, traversé par l'aiguille 3, est poussé par un ressort 41 pour maintenir en légère surpression l'huile isolante à l'intérieur du dispositif. Cette dernière permet le fonctionnement du dispositif dans un environnement à haute pression, au sein de fluides éventuellement corrosifs ou conducteurs.

Conformément aux figures 10 et 11, le potentiomètre 12 comprend, sur un support plan 12a en matière isolante, monté longitudinalement sur la pièce 4, trois pistes rectilignes 12b, 12c, 12d conductrices de l'électricité, disposées géométriquement en parallèle et électriquement en série. Sur ces pistes, parmi lesquelles les pistes extrêmes 12b 12d ont une résistivité quasiment nulle, la piste intermédiaire 12c formant la piste résistive du potentiomètre, frottent trois balais 12e fixes qui constituent les trois bornes du potentiomètre 12.

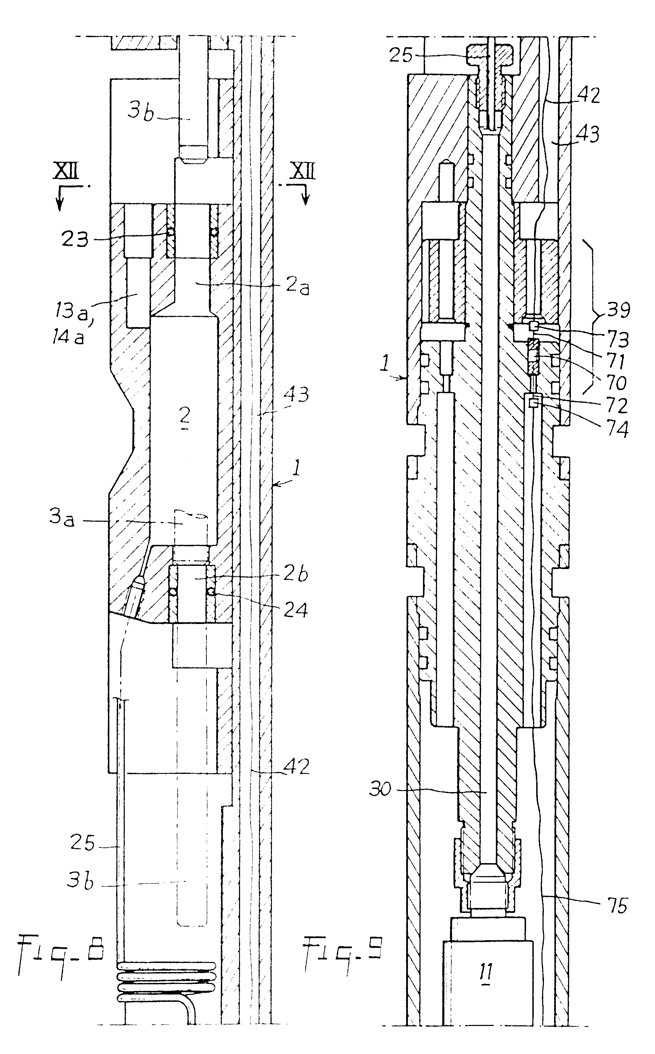

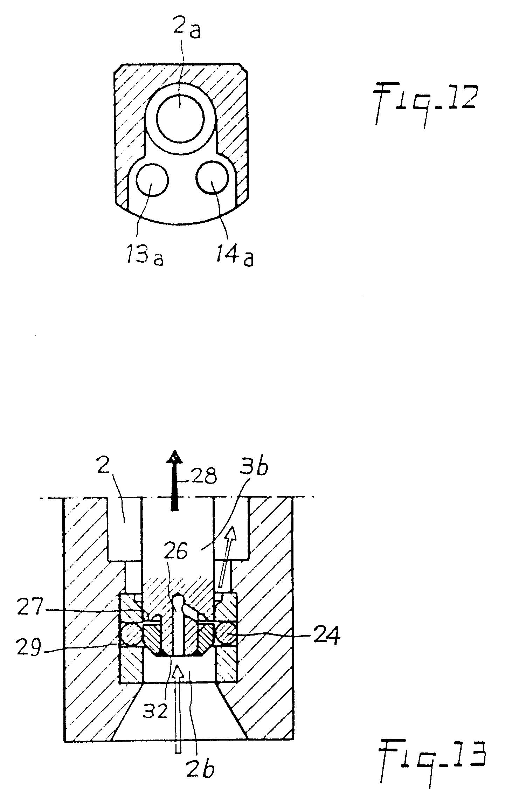

Par ailleurs, les figures 8 et 12 montrent que les deux valves 13, 14, susceptibles de mettre en communication l'intérieur de la chambre 2 avec le milieu extérieur, sont disposées d'un même côté de ladite chambre, dans des logements 13a, 14a creusés côte à côte dans l'une des extrémités de la paroi de la chambre.

Quant à la figure 13, elle fait voir la structure de l'extrémité de la partie terminale 3b de l'aiguille 3. On voit que dans cette extrémité est creusé un canal 26 débouchant d'une part dans la face frontale 32 par laquelle se termine l'aiguille et d'autre part dans une rainure périphérique 27 creusée dans la surface latérale de l'aiguille, à une distance de sa face frontale 32 légèrement supérieure à l'épaisseur du joint 24. Grâce à cette disposition, lorsque l'aiguille, remontant suivant la flèche 28, va quitter l'orifice 2b de la chambre 2 dans laquelle règne une pression P3 inférieure à la pression extérieure P1, la chambre se trouve reliée au milieu extérieur par un chemin (rainure 27 et canal 26) contournant le joint 24 alors que celui-ci est encore maintenu en place par l'aiguille. Ainsi, il ne risque pas d'être endommagé ou éjecté par une soudaine variation de pression de la valeur P3 à la valeur P1.

Un faisceau de fils électriques de connexion 42 traverse de part en part le dispositif vie un tunnel 43 prévu dans son enveloppe 1. La traversée étanche de la zone 39 (figure 9) comprend une traversée étanche proprement dite 70 terminée à ses deux extrémités par deux broches 71 et 72 connectées chacune à une prise respectivement 73 et 74. L'extrémité 75 du faisceau de fils électriques 42 est reliée à la partie électrique (non représentée) du capteur de pression 11, située sous ledit capteur. La chambre 2 est réalisée sous forme d'un élément indépendant à côté duquel passe ledit tunnel 43 (figure 8); elle peut être facilement détachée de l'enveloppe 1 pour permettre l'accès aux joints 24, sans qu'il soit besoin d'extraire le faisceau de fils électriques 42 traversant le dispositif.

A titre indicatif, dans un exemple de réalisation conforme à la description qui précède, on a choisi les spécifications techniques suivantes :
- longueur totale du dispositif : 2,75 m;
- pression et température admissibles dans le puits : environ 1 050 bar et 175 °C;
- volume de la chambre 2 : 12 cm$^3$;
- variation maximale du volume de la chambre 2 : 10 %.
- diamètres respectifs des parties 3a et 3b de l'aiguille 3 : 9 et 7,8 mm, d'où une variation de volume de la chambre de 13,5 mm$^3$ par mm de déplacement de l'aiguille;
- variation maximale de la pression dans la chambre 2 par rapport au milieu extérieur : environ ± 210 bar;
- gamme de mesure du manomètre 11 : environ 700 ou 1 400 bar.

Il est à observer qu'un dispositif tel que décrit ci-dessus est conçu exclusivement pour analyser les caractéristiques du pétrole au fond d'un puits par mesure de son point de bulle, et n'est pas adapté à faire remonter en surface des échantillons de pétrole prélevés au fond. Pour cette raison, il convient en pratique de combiner ce dispositif avec au moins un échantillonneur dans un ensemble à outils multiples.

Par ailleurs, on notera qu'un dispositif selon l'invention permet d'effectuer des mesures répétées autant de fois qu'on le désire durant une période de production du puits.

**Revendications**

1. Procédé de mesure du point de bulle du pétrole d'une formation souterraine dans laquelle a été foré un puits, comprenant les étapes suivantes : descendre dans le puits un outil comportant une chambre dont le volume peut varier sur commande; ouvrir la chambre de façon qu'elle se remplisse de pétrole; refermer la chambre et augmenter graduellement son volume en mesurant, pour chaque valeur de volume V imposée à la chambre, la pression P qui y règne; puis établir, à partir des points de mesure ainsi déterminés, la courbe P = f(V), d'où la position du point de bulle peut être déduite; caractérisé en ce que, après la fermeture de la chambre remplie de pétrole, on commande tout d'abord une diminution du volume de celle-ci de façon à y créer une surpression par rapport à la pression ambiante du pétrole dans le puits avant d'augmenter peu à peu le volume de la chambre en relevant les valeurs successives du volume et de la pression.

2. Dispositif de mesure du point de bulle du pétrole d'une formation souterraine dans laquelle a été foré un puits, comprenant un outil conçu pour être descendu dans le puits et comportant une chambre (2) à paroi rigide dont le volume peut varier grâce à un organe déplaçable en translation longitudinale sous l'action de moyens d'entraînement, la chambre (2), dont l'ouverture peut être commandée pour son remplissage étant reliée à un manomètre (11) mesurant la pression P qui y règne, et un capteur de déplacement (12) étant couplé audit organe (3), les signaux délivrés par le manomètre et le capteur de déplacement permettant de déterminer ledit point de bulle; caractérisé en ce que ladite chambre (2) comporte un orifice (2b) traversant ladite paroi et en ce que ledit organe est formé par une aiguille (3) susceptible d'être introduite dans ladite chambre au travers dudit orifice (2b) pour faire varier le volume de la chambre (2), ladite aiguille (3) pouvant ouvrir l'orifice (2b) pour le remplissage de la chambre.

3. Dispositif selon la revendication 2, caractérisé par le fait que l'aiguille (3) comporte deux parties successives (3a, 3b) de section différente, savoir une première partie (3a) qui se prolonge par une deuxième partie (3b) de section inférieure a celle de la première, et que la chambre (2) possède deux orifices (2a, 2b) alignés permettant à l'aiguille (3) de traverser de part en part la chambre, savoir un premier crifice (2a) de section conjuguée de celle de la première partie (3a) de l'aiguille et un deuxième orifice (2b) de section conjuguée de celle de la deuxième partie (3b), ces orifices étant obturés respectivement par lesdites parties de l'aiguille (3) lorsque celle-ci est amenée à traverser la chambre.

4. Dispositif selon la revendication 3, caractérisé par le fait que le moyen d'entraînement de l'aiguille (3) peut la faire se retirer complètement de la chambre (2) en libérant les deux orifices (2a, 2b).

5. Dispositif selon l'une quelconque ces revendications 2 à 4, caractérisé par le fait qu'une cremière valve tarée (13) de limitation de pression est disposée entre le volume intérieur ce la chambre (2) et la milieu extérieur, qui limite la surpression ΔP pouvant apparaître dans la chambre par repport à la pression P1 régnant dans le puits.

6. Dispositif selon l'une quelconque ces revendications 2 à 5, caractérisé par le fait qu'une deuxième valve tarée (14) ce limitation de pression est disposée entre le volume intérieur ce la chambre et le milieu extérieur, qui limite la dépression ΔP' pouvant apparaître dans la chambre par rapport à la pression P1 régnant dans le puits.

7. Dispositif selon l'une quelconque des revendications 2 à 6, caractérisé par le fait que les moyens d'entraînement de l'aiguille (3) comprennent un moteur électrique (7) logé dans un carter (15) étanche, à l'épreuve de la pression, une tige filatée (6) entraînée en rotation par le moteur (7) à travers la paroi de son carter (15) grâce à un dispositif d'accouplement magnétique (8), et un écrou (5) monté sur la tige filetée (6) et solidaire de l'aiguille (3).

8. Dispositif selon l'une quelconque des revendications 2 à 7, caractérisé par le fait que le capteur de déplacement de l'aiguille (3) est constitué par un potentiomètre linéaire (12).

9. Dispositif selon l'une quelconque des revendications 3 à 8, caractérisé par le fait que l'orifice (2b) de la chambre (2) avec lequel est susceptible de coopérer la deuxième partie (3b) de l'aiguille (3) est muni d'un joint annulaire d'étanchéité (24) et que dans l'extrémité de l'aiguille (3) est creusé un conduit (26) d'égalisation de pression mettant en communication, quand l'aiguille, au cours d'un mouvement de retrait de la chambre, va quitter ledit orifice (2b), la chambre (2) avec le milieu extérieur alors que l'extrémité de l'aiguille est encore engagée dans le joint (24).

**Claims**

1. A method of measuring the bubble point of oil in an underground formation into which a well has been drilled, the method comprising the steps of :

   lowering a tool down the well, said tool including a chamber whose volume is variable on command;

   opening the chamber so that it fills with oil;

   closing the chamber and increasing its volume gradually while measuring the pressure P inside the chamber for each value of volume V imposed thereon; then

   establishing the curve P = f(V) from the measurement points obtained in this way; whereby the position of the bubble can be deduced from said curve; characterized in that, once the chamber full of oil has been closed, the volume of the chamber is initially reduced so as to increase the pressure therein relative to the ambient pressure of oil in the well; and the volume of the chamber is then increased in stages with successive values of volume and pressure being taken.

2. Apparatus for measuring the bubble point of oil in an underground formation into which a well has been drilled, said apparatus comprising a tool designed to be lowered down the well and comprising :

   a rigid-walled chamber (2) whose volume is variable by a member longiturally movable in translation under the action of drive means, the chamber being capable of being opened for filling and being connected to a manometer (11) for measuring the pressure P inside said chamber;

   a needle insertable through at least said one orifice to vary the volume of said chamber;

   drive means for driving said needle in longitudinal translation;

   a displacement sensor (12) coupled to said member (3), the signals delivered by the manometer and the displacement sensor enabling the bubble point of said oil to be determined,

   characterized in that said chamber (2) has an orifice (2b) through said wall and in that said member is formed by a needle (3) insertable into said chamber through said orifice (2b) to vary the volume of the chamber (2), said needle being capable of opening the orifice (2b) for filling the chamber.

3. Apparatus according to claim 2, characterized in that said needle (3) comprises two successive portions (3a, 3b) of different cross-section, namely a first portion (3a) which is extended by means of a second portion (3b) of smaller cross-section than the first, and in that said chamber (2) includes two aligned orifices (2a, 2b) enabling said needle (3) to pass right through said chamber, namely a first orifice (2a) complementary in section to said first needle portion (3a) and a second orifice (2b) complementary in section to said second needle portion (3b), said orifices being closed by respective ones of said needle portions when said needle (3) is caused to pass through said chamber.

4. Apparatus according to claim 3, characterized in that the drive means of the needle (3) is suitable for withdrawing the needle sufficiently from said chamber (2) to open both orifice (2a, 2b).

5. Apparatus according to any one of claims 2 to 4, characterized in that a first pressure release valve (13), is disposed between the inside volume of the chamber (2) and the surrounding medium, to limit the pressure increase $\Delta P$ which may appear inside said chamber above the pressure P1 in the well.

6. Apparatus according to any one of claims 2 to 5, characterized in that a second pressure release valve (14) is disposed between the inside volume of the chamber and the surrounding medium to limit mesure decrease $\Delta P'$ which may appear inside said chamber below the pressure P1 in the well.

7. Apparatus according to any one of claims 2 to 6, characterized in that, the drive means of the needle (3) comprise an electric motor (7) housed in a pressure-proof housing (15), a threaded rod (6) rotated by the motor (7) through the wall of said housing (15) by means of a magnetic coupling (8), and a nut (5) mounted on the threaded rod (6) and fixed to the needle (3).

8. Apparatus according to any one of claims 2 to 7, characterized in that the displacement sensor of the needle (3) is constituted by a linear potentiometer (12).

9.  Apparatus according to any one of claims 3 to 8, characterized in that the orifice (2b) of the chamber (2) which co-operates with said second portion (3b) of the needle (3) is provided with an annular sealing ring (24), and wherein the end of said needle (3) has a pressure-equalizing duct (26) passing therethrough to put the chamber (2) into communication with the surrounding medium when the needle is about to leave said orifice (2b) as it is being withdrawn from the chamber but while the end of the needle is still engaged inside the sealing ring (24).

## Patentansprüche

1.  Verfahren zum Messen des Brodelpunktes von Erdöl einer unterirdischen Formation, in die ein Bohrloch abgeteuft worden ist, umfassend die folgenden Schritte: Absenken einer Sonde in das Bohrloch mit einer Kammer, deren Volumen gesteuert variierbar ist, Öffnen der Kammer derart, daß sie sich mit Erdöl füllt, Wiederverschließen der Kammer und graduelles Vergrößern ihres Volumens und Messen des in ihr herrschenden Druckes P für jedes der Kammer aufgezwungene Volumen V, Etablieren, ausgehend von den so bestimmten Meßpunkten, der Kurve P = f(V), aus der die Position des Brodelpunktes ableitbar ist, dadurch gekennzeichnet, daß nach Schließen der erdölgefüllten Kammer zuerst eine Verringerung des Volumens derselben bewirkt wird derart, daß ein Überdruck relativ zu dem Umgebungsdruck des Erdöls in dem Bohrloch erzeugt wird, bevor nach und nach das Volumen der Kammer unter Festhalten der sukzessiven Werte von Volumen und Druck vergrößert wird.

2.  Vorrichtung zum Messen des Brodelpunktes von Erdöl einer unterirdischen Formation, in die ein Bohrloch abgetsuft worden ist, umfassend eine in das Bohrloch ablaßbare Sonde mit einer Kammer (2) mit starrer Wandung, deren Volumen mittels eines unter der Wirkung von Antriebsmitteln in Längsrichtung translatorisch verlagerbaren Organs variierbar ist, welche Kammer (2), deren Öffnen für Ihre Füllung steuerbar ist, mit einem den in ihr herrschenden Druck messenden Manometer (11) verbunden ist, und einen mit dem Organ (3) verbundenen Verlagerungsfühler (2), wobei die von dem Manometer und dem Verlagerungsfühler gelieferten Signale die Bestimmung des Brodelpunktes ermöglichen, dadurch gekennzeichnet, daß die Kammer (2) eine die Wandung durchsetzende Öffnung (2b) aufweist und daß das Organ (3) als eine in die Kammer durch die Öffnung einführbare Nadel (3) zum Variieren des Volumens der Kammer (2) ausgebildet ist, welche Nadel (3) die Öffnung (2b) für das Füllen der Kammer öffnen kann.

3.  Vorrichtung nach Anspruch 2, dadurch gekennzeichnet, daß die Nadel (3) zwei aufeinanderfolgende Abschnitte (3a, 3b) unterschiedlichen Querschnitts aufweist, nämlich einen ersten Abschnitt (3a), der sich um einen zweiten Abschitt (3b) geringeren Querschnitts als der erste verlängert, und daß die Kammer (2) zwei ausgefluchtete Öffnungen (2a, 2b) besitzt, die es der Nadel (3) ermöglichen, die Kammer von einer zur anderen Seite zu durchsetzen, nämlich eine erste Öffnung (2a) eines zum Querschnitt des ersten Nadelabschnitts (3a) komplementären Querschnitts und eine zweite Öffnung (2b) eines zum Querschnitt des zweiten Nadelabschnitt (3b) komplementären Querschnitts, welche Öffnungen jeweils durch die Abschnitte der Nadel (3) gesperrt sind, wenn die Nadel zum Durchsetzen der Kammer gebracht ist.

4.  Vorrichtung nach Anspruch 3, dadurch gekennzeichnet, daß die Antriebsmittel der Nadel (3) diese vollständig aus der Kammer (2) zurückziehen können unter Freigabe der beiden Öffnungen (2a, 2b).

5.  Vorrichtung nach einem der Ansprüche 2 bis 4, dadurch gekennzeichnet, daß ein erstes tariertes Druckbegrenzungsventil (13) zwischen dem inneren Volumen der Kammer (2) und dem äußeren Milieu angeordnet ist, das den Überdruck P begrenzt, der in der Kammer relativ zu dem im Bohrloch herrschenden Druck P1 auftreten kann.

6.  Vorrichtung nach einem der Ansprüche 2 bis 5, dadurch gekennzeichnet, daß ein zweites tariertes Druckbegrenzungsventil (14) zwischen dem inneren Volumen der Kammer (2) und dem äußeren Milieu angeordnet ist, das den Unterdruck P' begrenzt, der in der Kammer relativ zu dem im Bohrloch herrschenden Druck P1 auftreten kann.

7.  Vorrichtung nach einem der Ansprüche 2 bis 6, dadurch gekennzeichnet, daß die Antriebsmittel der Nadel (3) einen Elektromotor (7) in einemn druckfest abgedichteten Gehäuse (15), eine von dem Motor (7) durch die Gehäusewandung hindurch mittels einer Magnetkupplung (8) zum Umlauf angetriebene Gewindespindel (6) und eine auf der Gewindespindel (6) angeordnete, mit der Nadel (3) verbundene Mutter (5) umfassen.

8.  Vorrichtung nach einem der Ansprüche 2 bis 7, dadurch gekennzeichnet, daß der Verlagerungsfühler der Nadel (3) von einem linearen Potentiometer (12) gebildet ist.

9.  Vorrichtung nach einem der Ansprüche 3 bis 8, dadurch gekennzeichnet, daß die Öffnung (2b) der Kammer (2), die mit dem zweiten Abschnitt (3b) der Nadel (3) in Wirkverbindung bringbar ist, mit einer Ringdichtung (24) versehen ist und daß in das Ende der Nadel (3) eine Druckausgleichsleitung (26) eingearbeitet ist, welche -- wenn die Nadel bei einer Rückzugsbewegung aus der Kammer an der Öffnung (2b) vorbeistreicht - - die Kammer mit dem äußeren Milieu in Kommunikation bringt, während das Nadelende noch in der Dichtung (24) sitzt.

Fig-1

Fig-2

Fig-3

Fig-4a

Fig-4b

Fig-4c

Fig-4d

*Fig-5*

*Fig-6*

Fig_7

Fig_10

Fig_11

Fig. 8

Fig. 9

2a

13a   14a

Fig. 12

2   28   3b

26

27

29   24

32

2b

Fig. 13